# EUROPÄISCHE PATENTSCHRIFT

(11) **EP 2 954 312 B1**
(45) Veröffentlichungstag und Bekanntmachung des Hinweises auf die Patenterteilung: **20.12.2017**
(21) Anmeldenummer: 14709564.0
(22) Anmeldetag: 07.02.2014
(51) Int. Cl.: G01N 21/78, C11D 3/39, C11D 7/10, C11D 7/12, C11D 7/16, C11D 11/00, C11D 3/12, C11D 7/20, G01N 31/22, G01N 21/77, G01N 33/52, A01N 59/02

(54) **DETEKTION VON ORGANISCHEN VERSCHMUTZUNGEN**
DETECTION OF ORGANIC IMPURITIES
DETECTION D'IMPURITEES ORGANIQUES

(30) Priorität: 07.02.2013 EP 13154384
(43) Veröffentlichungstag der Anmeldung: 16.12.2015
(73) Patentinhaber: Thonhauser GmbH, 2372 Gießhübl (AT)
(72) Erfinder: HERZOG, Daniel, A-3002 Purkersdorf (AT); THONHAUSER, Philip, A-2372 Gießhübl (AT)
(74) Vertreter: Ellmeyer, Wolfgang
(86) Internationale Anmeldenummer: PCT/EP2014/052461
(87) Internationale Veröffentlichungsnummer: WO 2014/122277

(56) Entgegenhaltungen:
- WO-A1-02/31098
- WO-A1-99/32596
- WO-A1-2005/044968
- WO-A2-02/23993
- DE-A1-102006 060 204
- GB-A- 1 510 452

## Beschreibung

Die Erfindung betrifft die Verwendung einer stark oxidierenden Zusammensetzung zum Detektieren etwaiger Verschmutzungen einer Oberfläche mit organischen Substanzen.

### STAND DER TECHNIK

Reinigungs- und Desinfektionsmittel, die starke Oxidationsmittel enthalten, sind seit Langem bekannt. Auch die Anmelderin beschreibt in älteren Patenten solche Zusammensetzungen. EP 1.343.864 B1 offenbart ein wasserlösliches Permanganat enthaltendes Reinigungs- und Desinfektionsmittel, das darüber hinaus einen pH-Regler zur Sicherstellung eines alkalischen Milieus von zumindest pH 10, vorzugsweise zumindest pH 12, wie z.B. NaOH, sowie zumindest ein weiteres Oxidationsmittel mit einem Oxidationspotenzial, das über jenem von Mangan(VII) zu Mangan(VI) und vorzugsweise über jenem von HO₂⁻ zu OH⁻ liegt, vorzugsweise ein Peroxodisulfat.

Eine solche Zusammensetzung entfaltet ihre Oxidationswirkung in stark alkalischem Milieu vorwiegend durch die Reduktion von Mn(VII) zu Mn(VI), wobei bei Gegenwart von organischem Kohlenstoff dieser geichzeitig zu Oxalat oxidiert wird. Das weitere Oxidationsmittel mit einem Oxidationspotenzial über jenem von Mangan(VII), wie z.B. ein oder mehrere Alkalimetallperoxodisulfate, reagiert dabei in der Regel langsamer mit organischem Kohlenstoff als Permanganat, so dass es bei Auflösung der pulverförmigen Zusammensetzung in Wasser zunächst zu einer Oxidation von Hydroxid-Ionen zu Wasserstoffperoxid-Ionen, und zwar sowohl durch das Peroxodisulfat als auch durch das Permanganat, das dabei zu Mn(VI) reduziert wird; siehe die nachstehenden Gleichungen 1 und 2:

Gl. 1: 3 OH⁻ + S₂O₈²⁻ → HO₂⁻ + 2 SO₄²⁻ + H₂O

Gl. 2: 4 OH⁻ + 4 MnO₄⁻ → O₂↑ + 4 MnO₄²⁻ + 2 H₂O

Das dabei entstehende Wasserstoffperoxid-Ion kann allerdings eine Reoxidation des Mn(VI) zu Mn(VII) bewirken:

Gl. 3: HO₂⁻ + 2 MnO₄²⁻ + H₂O → 3 OH⁻ + 2 MnO₄⁻

Wenn die Zersetzungsgeschwindigkeit des Peroxodisulfats mit jener des Permanganats nicht Schritt halten kann, z.B. weil die Zersetzung des Permanganats durch eine hohe Konzentration und/oder gute Oxidierbarkeit der organischen Verunreinigung begünstigt wird, kommt es zu vermehrter Bildung von Mn(VI). Die Dominanz der sechswertigen Mangan-Spezies führt zu einer grünen Färbung der Lösung, im Gegensatz zur anfänglichen violetten Färbung aufgrund von Mn(VII). Die Oxidation organischer Verbindungen (hier - stellvertretend für Kohlenstoff mit der Oxidationsstufe ±0 und im Speziellen für Kohlenhydrate - mit "CH₂O" bezeichnet) zu Oxalat durch Mn(VII) und die damit einhergehende Reduktion des Permanganats erfolgt jedenfalls rasch, da der hohe pH-Wert auf zahlreiche organische Stoffe anionisierend wirkt, was den Angriff von anionischen Oxidationsmitteln erleichtert. Die Oxidation organischer Substanzen durch Mn(VII) involviert dabei auch MnO₄³⁻, in dem Mangan mit der Oxidationszahl +5 vorliegt (Gl. 4), durch Permanganat aber wieder zu sechswertigem Mangan oxidiert werden kann (Gl. 5)

Gl. 4: 2 {CH₂O} + 3 MnO₄⁻ + 2 H₂O → C₂O₄²⁻ + 3 MnO₄³⁻ + 8 H⁺

Gl. 5: MnO₄³⁻ + MnO₄⁻ → 2 MnO₄²⁻

Der Angriff des Permanganats auf organische Substanzen gemäß Gl. 4 bedingt allerdings nicht die hohe Wirksamkeit einer solchen Kombination aus Permanganat und Peroxodisulfat. Die schnelle und effiziente Oxidation organischer Stoffe wird vielmehr durch nun startende Radikalreaktionen verursacht. Ausgangspunkt sind dabei SO₄⁻-Radikale, die aus dem Peroxodisulfat durch homolytische Spaltung von Peroxodisulfat (Gl. 6) oder durch dessen Reaktion mit organischen Verbindungen (Gl. 7) hervorgehen können (fettgedruckte Verbindungen kennzeichnen hierin Radikale oder Radikalionen):

Gl. 6: S₂O₈²⁻ → 2 **SO₄⁻**

Gl. 7: 2 S₂O₈²⁻ + 2 {CH₂O} + 2 H₂O → 2 SO₄²⁻ + 2 **SO₄⁻** + **{C⁺¹-R}** + 4 H⁺

**{C⁺¹-R}** steht dabei für ein Radikal mit Kohlenstoff in der Oxidationsstufe +1, z.B. formal **{H₂C₂O₃}²⁻,** bei dem zwischen den Kohlenstoff-Atomen eine Doppelbindung besteht.

Vorwiegend scheinen Sulfatradikale jedoch durch Reaktion von Mn(V) (siehe obige Gleichung 4) mit Peroxodisulfat zu entstehen (Gl. 8):

Gl. 8: MnO₄³⁻ + S₂O₈²⁻ → MnO₄²⁻ + SO₄²⁻ + **SO₄⁻**

Mn(V) wird dabei zu Mn(VI) reoxidiert. Aufgrund der thermodynamischen Instabilität von Mn(VI) bildet sich schließlich vorwiegend Mn(II):

Gl. 9: MnO₄²⁻ + H₂O → O₂↑ + HMnO₂⁻ + OH⁻

Dabei bewirkt das Mn(II) eine Gelbfärbung der Zusammensetzung. Eine solche Färbung kann auch als Hinweis darauf gedeutet werden kann, dass die Oxidationsmittel durch große Mengen an Verunreinigungen verbraucht wurden. Mn(II) wird durch Luftsauerstoff allmählich zu Mn(IV) oxidiert, das sich in der Folge als Braunstein, MnO₂, ablagern kann.

Kombinationen aus Permanganat und Peroxodisulfat zeigen somit synergistische Wirkung bei der Oxidation von organischem Kohlenstoff und stellen gleichzeitig ein Indikatorsystem bereit, da sich die ursprünglich violette Lösung in Gegenwart von organischem Kohlenstoff gut sichtbar grün verfärbt. Als mögliche Alternativen zu Peroxodisulfat werden Periodat, Peroxodiphosphat, Ozon und Hypochlorit erwähnt.

Gemäß EP 1.343.864 B1 kann das Reinigungs- und Desinfektionsmittel zur Reinigung von Getränke-Schankanlagen (mittels Durchspülen der Anlage mit den wässrigen Lösungen) oder verunreinigten Flaschen von organischen Rückständen oder auch zur Reinigung von Oberflächen in gemüseverarbeitenden Betrieben oder Brauereien eingesetzt werden, in letzterem Fall allerdings zur Entfernung von anorganischen Belägen - nach einer Einwirkzeit des Mittels auf die Beläge von "weniger als einer Stunde".

Eine Verbesserung des Reinigungs- und Desinfektionsmittels wird in EP 1.730.258 B1 offenbart, indem pH-Puffersubstanzen, vorzugsweise Alkalimetall(hydrogen)carbonate, sowie oxidationsbeständige Polyphosphate zugesetzt werden.

In EP 1.456.338 B1 offenbart die Anmelderin Kombinationen aus wasserlöslichem Chlorit und Bromat, die ebenfalls mittels eines pH-Reglers bei stark alkalischem pH stabilisiert sind, um die Bildung von Chlordioxid zu unterdrücken. Als Anwendungen werden erneut Getränke-Schankanlagen sowie Rohrleitungen in der Molkerei- und Getränkeindustrie und die Aufbereitung von Schwimmbarwasser genannt.

Ein Nachteil dieser Reinigungsmittel nach dem Stand der Technik liegt darin, dass die wässrigen Lösungen zumeist nur 1- bis 3%ige Lösungen des enthaltenen Oxidationsmittels sind. In Behältern oder Leitungen sind diese Lösungen ausgezeichnet einsetzbar, von Oberflächen fließen sie jedoch in der Regel rasch ab, wenn die Oberfläche keine exakt waagrechte Fläche, am besten noch mit erhöhtem Begrenzungsrand, ist. Speziell im Falle von glatten Edelstahloberflächen oder Keramikfliesen, wie sie in der Getränke- oder Lebensmittelindustrie weit verbreitet Anwendung finden, ist eine zuverlässige Reinigung von Oberflächen unmöglich, vor allem, wenn es sich um geneigte oder vertikale Oberflächen handelt. Einen weiteren Nachteil stellt die Braunsteinbildung dar, da Braunstein in Wasser unlöslich ist und mit den oben beschriebenen Mitteln kaum entfernbar ist.

Die vorliegende Erfindung hat zum Ziel, diese Nachteile zu beheben. Ein weiteres Ziel der Erfindung bestand darin, ein Verfahren zu entwickeln, mittels dessen eine visuelle Kontrolle des Verschmutzungsgrads von Industrieoberflächen ermöglicht wird.

### OFFENBARUNG DER ERFINDUNG

Diese Ziele erreicht die Erfindung durch Bereitstellung der neuen Verwendung einer Zusammensetzung auf wässriger Basis, die
a) zumindest ein starkes Oxidationsmittel,
b) ein Farbindikatorsystem und
c) ein oder mehrere Verdickungsmittel
umfasst, zum Detektieren etwaiger Verschmutzung einer Oberfläche mit organischen Substanzen mittels Sichtprüfung nach flächigem Auftrag der Zusammensetzung auf die Oberfläche, wobei
- die Zusammensetzung nach dem flächigen Auftrag aufgrund der Wirkung des Verdickungsmittels für eine vorbestimmte Zeitspanne im Wesentlichen nicht fließfähig ist;
- das oder die Verdickungsmittel während der vorbestimmten Zeitspanne in der Zusammensetzung stabil ist/sind; und
- das oder die Verdickungsmittel ausgewählt ist/sind aus synthetischen Schichtsilikaten, pyrogener Kieselsäure, Fettalkyl(benzol)sulfaten, -sulfonaten, -carboxylaten,
- phosphaten und -aminoxiden sowie Gemischen davon;
und wobei ein Farbumschlag der auf die Oberfläche aufgetragenen Zusammensetzung während der vorbestimmten Zeitspanne organische Verschmutzungen der Oberfläche an dieser Stelle anzeigt.

Die Erfindung beruht auf mehreren überraschenden Entdeckungen der Erfinder: Erstens, dass der Zusatz eines Verdickungsmittels zu einer zumindest ein starkes Oxidationsmittel und ein Farbindikatorsystem umfassenden, wässrigen Zusammensetzung nicht nur seiner Bestimmung gemäß die Zusammensetzung eindickt und damit die Fließfähigkeit einschränkt, sondern auch eine stabilisierende Wirkung auf das Oxidationsmittel und damit auf die Farbentwicklung hat. Und zweitens, dass als Verdickungsmittel nicht nur anorganische, sondern auch organische Verdicker, wie z.B. organische Tenside, einsetzbar sind, obwohl das zumindest eine starke Oxidationsmittel organische Verunreinigungen sehr rasch oxidieren soll, wie dies später näher ausgeführt wird.

Ohne sich auf eine bestimmte Theorie festlegen zu wollen, wird angenommen, dass die stabilisierende Wirkung auf das Oxidationsmittel auf der eingeschränkten Mobilität der Ionen in der verdickten Lösung und dem gleichzeitig eingeschränkten Zutritt von Luftsauerstoff beruht. Bei gemäß vorliegender Erfindung bevorzugter Verwendung von Permanganat als Oxidationsmittel werden dadurch sowohl die Bildung von Mn(II)-Spezies und damit die Gelbfärbung der Zusammensetzung als auch die in der Folge auftretende Oxidation von Mn(II) zu Mn(IV) unterdrückt. Der Verdicker stabilisiert somit einerseits die Färbung der Zusammensetzung - violett bei Abwesenheit organischer Verunreinigungen, grün in deren Gegenwart - und verringert gleichzeitig die Bildung von Braunsteinablagerungen, die mit wässrigen Lösungen schwer zu entfernen sind.

Die Art und Menge des Verdickungsmittels werden vorzugsweise so gewählt, dass der Zusammensetzung zumindest für die vorbestimmte Zeitspanne eine Konsistenz verliehen wird, die bewirkt, dass sie selbst von geneigten, gekrümmten oder vertikalen Flächen, wie z.B. Rohren oder Wänden, oder sogar abwärts weisenden Oberflächen, wie z.B. Deckenfliesen oder dergleichen, im Wesentlichen nicht abfließt oder davon herabtropft.

Unter "im Wesentlichen nicht fließfähig" ist hierin eine Konsistenz der Zusammensetzung gemeint, bei der diese so stark eingedickt ist, dass eine auf eine vertikale Oberfläche flächig aufgetragene Schicht der Zusammensetzung während der vorbestimmten Zeitspanne um nicht mehr als 10 cm, vorzugsweise nicht mehr als 5 cm, insbesondere nicht mehr als 2 cm abwärts rinnt. Dadurch ist es gemäß vorliegender Erfindung nicht nur möglich, die Gegenwart, sondern auch die Position von Verunreinigungen auf der zu prüfenden Oberfläche sehr exakt zu bestimmen.

Die vorbestimmte Zeitspanne hängt zwar unter anderem von der Konzentration des oder der Oxidationsmittel sowie von der Art des Verdickungsmittels - anorganisch oder organisch oder beides - ab, beträgt aber vorzugsweise zumindest 10 s, noch bevorzugter zumindest 20 s, noch bevorzugter zumindest 15 s, noch bevorzugter zumindest 1 min, um während dieser Zeitspanne alle möglicherweise auf der Oberfläche vorhandenen Verunreinigungen oxidieren zu können.

Das zumindest eine Oxidationsmittel wird dabei vorzugsweise aus Permanganaten, Peroxodisulfaten, Salzen von Halogensauerstoffsäuren und Gemischen davon ausgewählt, bei denen es sich durchwegs um starke Oxidationsmittel handelt, die in der Lage sind, organische Verunreinigungen sehr rasch zu oxidieren. In besonders bevorzugten Ausführungsformen umfasst das Oxidationsmittel Permanganat, da dessen Verwendung gleichzeitig ein Farbindikatorsystem bereitstellt, zu welchem Zweck das Permanganat in einer Mindestmenge von 0,1 g/l in der Zusammensetzung enthalten sein sollte, um den Farbumschlag gut detektieren zu können. Bei Verwendung anderer Oxidationsmittel als Permanganat steht dem Fachmann jedoch auch eine ganze Reihe einsetzbarer Indikatorsysteme zur Verfügung, wie z.B. Kaliumiodid, Dichromat oder Dichlorphenolindophenol im Kombination mit Wasserstoffperoxid oder Ferroin für Persulfat.

Vorzugsweise umfasst die Zusammensetzung neben Permanganat zumindest ein zusätzliches Oxidationsmittel, dessen Oxidationspotenzial über jenem von Permanganat liegt, um die eingangs beschriebenen Reoxidations- und Synergieeffekte nutzen zu können. Das zusätzliche Oxidationsmittel ist dabei besonders bevorzugt aus Peroxodisulfat, Hypochlorit und Gemischen davon ausgewählt und wird in besonders bevorzugten Ausführungsformen der Erfindung als Hauptoxidationsmittel eingesetzt, d.h. es ist in einer größeren Menge als das Permanganat in der Zusammensetzung enthalten, die vorzugsweise zumindest das 10fache, noch bevorzugter zumindest das 20fache, insbesondere zumindest das 30fache der Menge an Permanganat, und vorzugsweise zumindest 0,3 g/l beträgt, wie dies aus den späteren Beispielen hervorgeht.

Wie oben beschrieben, ist das oder sind die Verdickungsmittel ausgewählt aus synthetischen Schichtsilikaten, pyrogener Kieselsäure, Fettalkyl(benzol)sulfaten, -sulfonaten, -carboxylaten, -phosphaten und -aminoxiden sowie Gemischen davon, da die Erfinder im Zuge ihrer Forschungen überraschenderweise festgestellt haben, dass:
a) natürliche anorganische Verdicker, wie z.B. Bentonit, die offenbar nicht zu vernachlässigende Mengen an organischen Verunreinigungen enthalten, oder organisch modifizierte natürliche Verdicker, wie z.B. diverse Optigel®-Verdicker, während der erfindungsgemäßen Verwendung nicht stabil sind, sondern von dem zumindest einen Oxidationsmittel angegriffen werden; obwohl
b) bestimmte organische Verdicker, namentlich Fettalkyl(benzol)sulfate, -sulfonate, -carboxylate, -phosphate und -aminoxide, im Gegensatz zu den meisten anderen organischen Verdickungsmitteln sehr wohl ausreichende Stabilität für die erfindungsgemäße Verwendung zeigen, da offenbar langkettige Alkyl- sowie Phenylgruppen - zumindest für die vorbestimmte Zeitspanne - hinreichend oxidationsresistent sind.

Diese organischen Verdickungsmittel sind vorzugsweise in einer Gesamtmenge von nicht mehr als 3 Gew.-%, noch bevorzugter nicht mehr als 2 Gew.-%, insbesondere nicht mehr als 1 Gew.-%, der gesamten Zusammensetzung enthalten, um auch vor Gebrauch Stabilität zu gewährleisten.

Kombinationen aus anorganischen und organischen Verdickungsmitteln, z.B. aus einem Schichtsilikat und einem organischen Tensid, haben beispielsweise bei Verwendung von Permanganat als Oxidationsmittel - neben der gemeinsam ausgeübten Verdickungswirkung - den Vorteil, dass der anorganische Verdicker hauptverantwortlich für die stabilisierende Wirkung auf die Mangan-Spezies sein kann, während das organische Tensid dafür sorgt, dass sich etwaiger dennoch gebildeter Braunstein leichter von der inspizierten Oberfläche entfernen lässt.

Vorzugsweise umfasst die Zusammensetzung weiters ein oder mehrere Hilfsmittel, die besonders bevorzugt aus pH-Reglern, Härtestabilisatoren und Bioziden ausgewählt sind. Beispielsweise ist es bei Verwendung mancher Oxidationsmittel oder Kombinationen davon erforderlich, den pH der Zusammensetzung auf stark alkalische Werte, z.B. von zumindest 10 oder 11, einzustellen, zu welchem Zweck besonders bevorzugt NaOH oder KOH eingesetzt werden. Zwar können auch andere starke Basen verwendet werden, die jedoch aus Kostengründen nicht bevorzugt werden.

In bevorzugten Ausführungsformen der Erfindung wird die Oberfläche durch den flächigen Auftrag der Zusammensetzung gleichzeitig gereinigt, d.h. nach der Sichtprüfung, ob und wo die Oberfläche verschmutzt ist, kann die Zusammensetzung - gegebenenfalls nach einer zusätzlichen, über die vorbestimmte Zeitspanne hinausgehenden Einwirkdauer - zusammen mit sämtlichen organischen oder anorganischen Rückständen entfernt werden, was vorzugsweise durch einfaches Abspülen der Oberfläche mit klarem Wasser erfolgt, gegebenenfalls gefolgt vom Abwischen der Oberfläche, z.B. mit feuchten Tüchern oder Abziehern.

Die Sichtprüfung kann dabei einfach mit bloßem Auge erfolgen, wenn der verwendete Farbindikator einen ausreichend unterscheidbaren Farbumschlag zeigt, kann aber auch computerunterstützt unter Durchführung digitaler Farbvergleiche durchgeführt werden. Letzteres ist beispielsweise zu bevorzugen, wenn der Verschmutzungsgrad der zu prüfenden Oberfläche sehr gering ist und daher nur eine schwache Farbveränderung des Indikators erfolgt. Zu diesem Zweck kann die zu prüfende Oberfläche nach dem flächigen Auftrag der Zusammensetzung und Verstreichenlassen einer ausreichenden Einwirkdauer mit einer Digitalkamera fotografiert werden und das Foto mittels einer Farbvergleichssoftware mit einer vorgegebenen Farbskala verglichen werden, z.B. unter Konvertierung der Farben auf dem Foto, oder zumindest auf einem Ausschnitt desselben, in RGB-Werte.

### KURZBESCHREIBUNG DER ZEICHNUNG

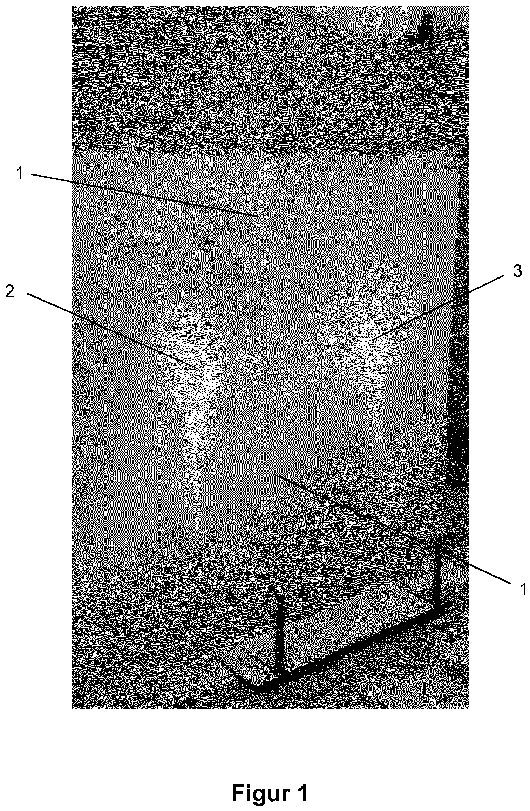

Fig. 1 als einzige Zeichnung ist ein Foto, welches das Ergebnis eines Detektionstests gemäß Beispiel 40 der vorliegenden Erfindung auf einer gezielt verunreinigten, vertikalen Edelstahloberfläche zeigt.

Nachstehend wird die Erfindung anhand von konkreten, nichteinschränkenden Ausführungs- und Vergleichsbeispielen näher beschrieben.

### BEISPIELE

Zusammensetzungen zur Verwendung gemäß vorliegender Erfindung wurden hergestellt, indem Kaliumpermanganat und ein zweites starkes Oxidationsmittel, ausgewählt aus Natriumhypochlorit (HC) und Natriumperoxodisulfat (PS) zusammen mit jeweils einem handelsüblichen anorganischen oder organischen Verdickungsmittel (VD) in den folgenden Anteilen unter Rühren in Wasser gelöst wurden:

| | |
|---|---|
| KMnO₄ | 0,01 g |
| HC oder PS | 0,12 g |
| NaOH | 0,04 g |
| VD | 0,5 bis 7 g |
| Wasser | auf 100 ml |

### Beispiele 1 bis 38, Vergleichsbeispiele 1 bis 82 - Stabilitätstests

Wie oben hergestellte Lösungen wurden in einem Photoreader vermessen, wobei untersucht wurde, ob das Verhältnis zwischen Mn(VII) und Mn(VI) über einen Zeitraum von 20 min über 75 % lag. Anschließend wurden 0,5 ml einer 0,1%igen Glucoselösung zugegeben, und der Farbverlauf wurde über einen Zeitraum von 10 min verfolgt. Eine Zusammensetzung wurde als stabil angesehen, wenn das Verhältnis Mn(VII)/Mn(VI) über 75 % lag und ein eindeutiger Farbumschlag von Violett zu Grün erfolgte.

Die genauen Mengen der jeweiligen Verdickungsmittel sind zusammen mit der Bewertung "+" für stabil oder "-" für nicht stabil in den nachstehenden Tabellen 1 und 2 angegeben, wobei in den Beispielen 1 bis 34 und Vergleichsbeispielen 1 bis 72 aus Tabelle 1 organische Verdickungsmittel und in den Beispielen 35 bis 38 und den Vergleichsbeispielen 73 bis 82 aus Tabelle 2 anorganische Verdickungsmittel getestet wurden. In den Tabellen sind von den einzelnen Verdickungsmitteln ihre Handelsnamen, der Hersteller und, soweit bekannt, die Wirksubstanz oder zumindest deren Substanzklasse angeführt. Gänzlich unbekannt waren in den allermeisten Fällen aber die sonstigen Begleitstoffe der handelsüblichen Produkte, da diesbezügliche Angaben der Hersteller im Normalfall fehlen.

Aus diesem Grund lassen sich auch keine allgemeingültigen Angaben bezüglich der Eignung handelsüblicher Produkte für die vorliegende Erfindung machen. Eine detaillierte Interpretation der erzeilten Ergebnisse findet sich freilich im Anschluss an die Tabellen.

Die Handelsnamen der Verdickungsmittel sind großteils markengeschützt. Auf die Kennzeichnung ® oder ™ in jedem Einzelfall wurde jedoch der Einfachkeit halber in der Folge verzichtet.

**Tabelle 1 - organische Verdickungsmittel**

| **Beispiel Nr.** | **Verdickungsmittel** | **Hersteller** | **Wirksubstanz** | **Ox. 2** | **VD (Gew.-%)** | **Bewertung** |
|---|---|---|---|---|---|---|
| Beispiel 1 | Dowfax 2A1 | Dow | verzweigtes C12-Alkyldiphenyloxiddisulfonat | PS | 2 | + |
| V1 | -"- | -"- | -"- | -"- | 7 | - |
| Beispiel 2 | -"- | -"- | - " - | HC | 2 | + |
| V2 | -"- | -"- | -"- | -"- | 7 | - |
| Beispiel 3 | Dowfax 3B2 | -"- | lineares C10-Alkyldiphenylsulfonat | PS | 2 | + |
| Beispiel 4 | -"- | -"- | -"- | -"- | 7 | + |
| Beispiel 5 | -"- | -"- | -"- | HC | 2 | + |
| Beispiel 6 | -"- | -"- | -"- | -"- | 7 | + |
| V3 | Methocel 267 | -"- | Celluloseether | PS | 1 | - |
| V4 | -"- | -"- | -"- | HC | 1 | - |
| V5 | Rhodopol 23 | Rhodia | Xanthangummi | PS | 1 | - |
| V6 | -"- | -"- | -"- | HC | 1 | - |
| Beispiel 7 | Genaminox LA | Clariant | Lauryldimethylaminoxid | HC | 2 | + |
| V7 | -"- | -"- | -"- | -"- | 7 | - |
| Beispiel 8 | Genaminox CSL | -"- | Kokosalkyldimethylaminoxid | PS | 2 | + |
| V8 | -"- | -"- | -"- | -"- | 7 | - |
| V9 | -"- | -"- | -"- | HC | 2 | - |
| V10 | -"- | -"- | -"- | -"- | 7 | - |
| V11 | Genamin CC 302D | -"- | Kokosalkyld imethylamin | HC | 0,5 | - |
| V12 | -"- | -"- | -"- | -"- | 3 | - |
| V13 | Sandoperm ACN | -"- | aminomodifiziertes Silikonelastomer | -"- | 0,5 | - |
| V14 | -"- | -"- | -"- | -"- | 3 | - |
| Beispiel 9 | Akyposoft 100 BVC | KAO | Polyoxyethylen-(11)-laurylethercarboxylat-Na | PS | 2 | + |
| Beispiel 10 | -"- | -"- | -"- | -"- | 7 | + |
| V15 | -"- | -"- | -"- | HC | 2 | - |
| V16 | -"- | -"- | -"- | -"- | 7 | - |
| Beispiel 11 | Oxidet DM-20 | -"- | Dimethyllauraminoxid | PS | 2 | + |
| Beispiel 12 | -"- | -"- | -"- | -"- | 7 | + |
| Beispiel 13 | -"- | -"- | - " - | HC | 2 | + |
| Beispiel 14 | -"- | -"- | -"- | -"- | 7 | + |
| Beispiel 15 | Oxidet L-75 C | -"- | Kokosalkylamidopropylaminoxid | PS | 2 | + |
| V17 | -"- | -"- | -"- | -"- | 7 | - |
| V18 | -"- | -"- | -"- | HC | 2 | - |
| V19 | -"- | -"- | -"- | -"- | 7 | - |
| V20 | Amidet A-15 | -"- | Polyoxyethylen-(2)-tridecylethercarbonsäureethanolamid | HC | 0,5 | - |
| V21 | -"- | -"- | -"- | -"- | 3 | - |
| V22 | Betadet HR-50K | -"- | Kokosalkylamidopropylbetain | HC | 2 | - |
| V23 | -"- | -"- | -"- | -"- | 7 | - |
| V24 | Betadet S-20 | -"- | Laurylhydroxysulfobetain | PS | 2 | - |
| V25 | -"- | -"- | -"- | -"- | 7 | - |
| V26 | -"- | -"- | -"- | HC | 2 | - |
| V27 | -"- | -"- | -"- | -"- | 7 | - |
| V28 | Betadet SHR | -"- | Kokosalkylamidopropylhydroxysulfobetain | PS | 2 | - |
| V29 | -"- | -"- | -"- | -"- | 7 | - |
| V30 | -"- | -"- | -"- | HC | 2 | - |
| V31 | -"- | -"- | -"- | -"- | 7 | - |
| Beispiel 16 | Quartamin AB | -"- | Behenyltrimethylammoniumchlorid | HC | 2 | + |
| Beispiel 17 | -"- | -"- | -"- | -"- | 7 | + |
| Beispiel 18 | Emal 30E | -"- | Natriumlaurylsulfat | PS | 0,5 | + |
| Beispiel 19 | -"- | -"- | -"- | -"- | 2 | + |
| V32 | -"- | -"- | -"- | -"- | 3 | - |
| V33 | -"- | -"- | -"- | -"- | 7 | - |
| V34 | -"- | -"- | -"- | HC | 2 | - |
| V35 | -"- | -"- | -"- | -"- | 7 | - |
| V36 | Emal 10N | -"- | Natriumlaurylsulfat | HC | 0,5 | - |
| V37 | -"- | -"- | -"- | -"- | 3 | - |
| V38 | Servo AM 1010 | Elementis | Kokosalkylaminopropionat | HC | 0,5 | - |
| V39 | -"- | -"- | -"- | -"- | 3 | - |
| V40 | Serdox NBS 6.6/90 | -"- | Polyoxyethylen-(6,6)-C₉₋₁₁-alkylether | PS | 2 | - |
| V41 | -"- | -"- | -"- | -"- | 7 | - |
| V42 | -"- | -"- | -"- | HC | 2 | - |
| V43 | -"- | -"- | -"- | -"- | 7 | - |
| V44 | Serdox NBSQ 5/5 | -"- | Polyoxyethylen-(5)-polyoxypropylen-(5)-C₉₋₁₁-alkylether | PS | 2 | - |
| V45 | -"- | -"- | -"- | -"- | 7 | - |
| V46 | Sermul EA 266 | -"- | Polyoxyethylen-(15)-tridecylethersulfat-Na | PS | 2 | - |
| V47 | -"- | -"- | -"- | -"- | 7 | - |
| V48 | -"- | -"- | -"- | HC | 2 | - |
| V49 | -"- | -"- | -"- | -"- | 7 | - |
| V50 | Servo XB 90 | -"- | Gemisch aus (quat. Ammonium-) kation. u. anion. Tensiden | PS | 2 | - |
| V51 | -"- | -"- | -"- | -"- | 7 | - |
| V52 | Servo XB 90 | -"- | Gemisch aus (quat. Ammonium-) kation. u. anion. Tensiden | HC | 2 | - |
| V53 | -"- | -"- | -"- | -"- | 7 | - |
| V54 | Servo Q 8010 | -"- | Laurylamidopropyltrimethylammoniummethylsulfat | PS | 2 | - |
| V55 | -"- | -"- | -"- | -"- | 7 | - |
| V56 | -"- | -"- | -"- | HC | 2 | - |
| V57 | -"- | -"- | -"- | -"- | 7 | - |
| Beispiel 20 | Crodasinic LS30 NP | Croda | Natriumfettacylsarcosinat | PS | 2 | + |
| V58 | -"- | -"- | -"- | -"- | 7 | - |
| Beispiel 21 | -"- | -"- | -"- | HC | 2 | + |
| V59 | -"- | -"- | -"- | -"- | 7 | - |
| Beispiel 22 | Multitrope 1214 | -"- | Fettalkylphosphatester | HC | 0,5 | + |
| Beispiel 23 | -"- | -"- | -"- | -"- | 3 | + |
| Beispiel 24 | Poro TS 430X | Poro | | PS | 2 | + |
| Beispiel 25 | -"- | -"- | -"- | -"- | 7 | + |
| V60 | -"- | -"- | -"- | HC | 2 | - |
| V61 | -"- | -"- | -"- | -"- | 7 | - |
| Beispiel 26 | Hoesch L29 | J. Hoesch | Polyoxyethylen-(3)-laurylethersulfosuccinat-2Na | HC | 0,5 | + |
| V62 | -"- | -"- | -"- | -"- | 3 | - |
| Beispiel 27 | Plurafac LF 221 | BASF | Polyalkylenoxidfettalkylether | PS | 2 | + |
| V63 | -"- | -"- | -"- | -"- | 7 | - |
| V64 | -"- | -"- | -"- | HC | 2 | - |
| V65 | -"- | -"- | -"- | -"- | 7 | - |
| V66 | Lutensol GD 70 | -"- | Fettalkylpolyglucosid | HC | 2 | - |
| V67 | -"- | -"- | -"- | -"- | 7 | - |
| Beispiel 28 | Lutensol TO 89 | -"- | Polyoxyethylen-C₁₃-isoalkylether | PS | 2 | + |
| Beispiel 29 | -"- | -"- | -"- | -"- | 7 | + |
| V68 | -"- | -"- | -"- | HC | 2 | - |
| V69 | -"- | -"- | -"- | -"- | 7 | - |
| Beispiel 30 | Glanapon DA 363 | Bussetti | modifiziertes Silikon | PS | 0,5 | + |
| V70 | -"- | -"- | -"- | -"- | 3 | - |
| V71 | Acticide PHB 20 | Thor | Polyhexamethylenbiguanid | HC | 0,5 | - |
| V72 | -"- | -"- | -"- | -"- | 3 | - |
| Beispiel 31 | Ammonyx LO | Stepan | Dodecanaminoxid | PS | 0,5 | + |
| Beispiel 32 | -"- | -"- | -"- | -"- | 3 | + |
| Beispiel 33 | -"- | -"- | -"- | HC | 0,5 | + |
| Beispiel 34 | -"- | -"- | -"- | -"- | 3 | + |

**Tabelle 2 - anorganische Verdickungsmittel**

| **Beispiel** N r. | **Verdickungsmittel** | **Hersteller** | **Wirksubstanz** | **Ox. 2** | VD **(Gew.-%)** | **Bewertung** |
|---|---|---|---|---|---|---|
| V73 | Laponite EP | Rockwood | organisch modifiziertes natürliches Schichtsilikat | PS | 1 | - |
| V74 | -"- | -"- | -"- | HC | 1 | - |
| Beispiel 35 | Laponite RD | -"- | synthetisches Schichtsilikat | PS | 1 | + |
| Beispiel 36 | -"- | -"- | -"- | HC | 1 | + |
| V75 | Optigel CK | -"- | natürlicher Bentonit | PS | 1 | - |
| V76 | -"- | -"- | -"- | HC | 1 | - |
| V77 | Optigel WX | -"- | organisch modifiziertes natürliches Schichtsilikat | PS | 1 | - |
| V78 | -"- | -"- | -"- | HC | 1 | - |
| V79 | Optigel W724 | -"- | organisch modifizierter Smectit | PS | 1 | - |
| V80 | -"- | -"- | -"- | HC | 1 | - |
| V81 | Optigel WA | -"- | aktivierter Smectit | PS | 1 | - |
| V82 | -"- | -"- | -"- | HC | 1 | - |
| Beispiel 37 | HDK D1515B | Wacker | pyrogene Kieselsäure | PS | 1 | + |
| Beispiel 38 | -"- | -"- | -"- | HC | 1 | + |

Ohne sich auf bestimmte Theorien festlegen zu wollen und mit der zuvor erwähnten Einschränkung, dass die Begleitstoffe der zu den obigen handelsüblichen Produkten formulierten Wirksubstanzen unbekannt waren, kann aus obiger Tabelle 1 Folgendes abgeleitet werden:

Tendenziell ist Peroxodisulfat (PS) als zweites starkes Oxidationsmittel gegenüber Hypochlorit (HC) zu bevorzugen, wenn organische Tenside als Verdickungsmittel zum Einsatz kommen sollen. Weiters ist die Einsatzmenge der organischen Tenside eher gering zu halten, um die Stabilität der Zusammensetzung für eine ausreichende Zeitspanne zu gewährleisten. Obgleich manche Tenside auch in einem Anteil von 7 Gew.-% stabil blieben, bewirkten andere schon bei über 2 Gew.-% eine Reaktion. Die Konzentration des organischen Tensids in der Zusammensetzung sollte daher vorzugsweise nicht mehr als 3 Gew.-%, noch bevorzugter nicht mehr als 2 Gew.-%, betragen. Falls diese Menge für die gewünschte Einschränkung der Fließfähigkeit der Zusammensetzung nicht ausreicht, können zusätzlich ein oder mehrere anorganische Verdickungsmittel zugesetzt werden, was außerdem, wie oben erwähnt, Vorteile bei der Entfernung anorganischer Rückstände mit sich bringt.

Was die Stoffklassen der organischen Tenside betrifft, sind anionische Tenside wie Fettalkyl(benzol)sulfate, -sulfonate, -carboxylate und -phosphate tendenziell stabiler als kationische Tenside, wie z.B. Fettalkylammoniumsalze, oder (in stark alkalischem Milieu) neutrale Moleküle wie Fettalkylamine, amphotere Tenside (Betaine) oder nichtionogene Tenside. Letztere, wie z.B. Polyalkylenoxidfettalkylether, waren zwar mitunter stabil, sollten aber besser mit anionischen Gruppen modifiziert werden. Eine Ausnahme bilden Fettalkylaminoxide, die sehr gute Ergebnisse geliefert haben. Polysaccharide wie Cellulose- und Xanthan-Derivate waren erwartungsgemäß nicht stabil, was auf die leichte Oxidierbarkeit der zahlreichen OH-Gruppen zurückzuführen sein dürfte. Von den beiden getesteten Silikonderivaten war eines in geringer Menge geeignet, dessen genaue Modifikation allerdings nicht bekannt war. Das andere, ein aminomodifiziertes Silikonelastomer, war hingegen nicht einmal in einer Menge von 0,5 Gew.-% stabil.

Tabelle 2 mit den mit anorganischen Verdickungsmitteln erzielten Ergebnissen zeigt, dass sowohl synthetisches Schichtsilikat als auch pyrogene Kieselsäure (auch als amorphe Kieselsäure bezeichnet) für die Verwendung als Verdickungsmittel in der Zusammensetzung geeignet sind. Auch hier hängt es im Einzelnen von der Modifikation ab, ob eine ausreichend stabile Zusammensetzung erhalten werden kann oder nicht. Ohne sich auf eine bestimmte Theorie festlegen zu wollen, wird angenommen, dass natürliche Produkte zu große Mengen an organischen Verunreinigungen enthalten, die von den Oxidationsmitteln angegriffen werden und zu falschen Ergebnissen bei der Detektion führen. Selbiges gilt in umso stärkerem Maße für organisch modifizierte anorganische Verdickungsmittel.

Ein durchschnittlicher Fachmann ist auf Basis seines allgemeinen Fachwissens und unter Zuhilfenahme der Anleitungen hierin problemlos und ohne übermäßiges Experimentieren, sondern durch einfache Reihenversuche in der Lage, ein geeignetes synthetisch-anorganisches und/oder ein unter die erfindungsgemäße Auswahl fallendes organisches Verdickungsmittel für eine ansonsten vorbestimmte Zusammensetzung zu ermitteln.

### Beispiele 39 bis 42, Vergleichsbeispiel 83 - vertikale Oberfläche

Zusammensetzungen mit den Verdickungsmitteln Dowfax 2A1 (analog zu Beispiel 1, aber mit 0,5 Gew.-% Verdickungsmittel), Ammonyx LO (analog zu Beispiel 33), Laponite RD (analog zu Beispiel 35, aber mit 1,5 Gew.-% Verdickungsmittel) und HDK D1515B (analog zu Beispiel 37, aber mit 1,5 Gew.-% Verdickungsmittel), d.h. zwei organischen (Fettalkyldiphenyloxiddisulfonat; Dodecanaminoxid) und zwei anorganischen Verdickungsmitteln (Schichtsilikat; pyrogene Kieselsäure), wurden als Zusammensetzungen der Beispiele 39 bis 42 hergestellt. Eine entsprechende Zusammensetzung ohne Verdickungsmittel mit Peroxodisulfat als zweites Oxidationsmittel diente als Vergleichsbeispiel 83.

Alle vier Zusammensetzungen wurden auf eine vertikale Edelstahloberfläche aufgesprüht, auf die zuvor mit einer Sprühlasche ein Hub einer 0,1%igen bzw. 0,01%igen Glucoselösung aufgebracht und getrocknet worden war, um eine Glucosemenge von etwa 1 mg/100cm² bzw. 0,1 mg/100cm² als organische "Verunreinigung" der Oberfläche zu erhalten.

Die Zusammensetzungen aus Beispiel 39 (Dowfax 2A1) und Beispiel 40 (Ammonyx LO) lagen dabei jeweils als stabiler Schaum, jene aus Beispiel 41 (Laponite RD) als thixotropes Gel und jene aus Beispiel 42 (HDK D1515B) als thixotrope Flüssigkeit vor.

Die anfangs violett gefärbten Zusammensetzungen der Beispiele 39 bis 42 verfärbten sich auf den verunreinigten Stellen der Oberfläche binnen 30-120 s grün, was die Gegenwart der organischen Verunreinigung anzeigte. Die grüne Färbung wurde danach jeweils noch 1-2 min lang beibehalten, ohne dass eine merkliche Verlagerung der Zusammensetzung durch Abfließen festzustellen war.

Bei der Zusammensetzung aus Vergleichsbeispiel 83 ohne Verdickungsmittel war kaum eine merkliche Verfärbung festzustellen, da sie von der Oberfläche sofort abfloss. Aufgrund der somit geringen Schichtdicke war zudem keinerlei Farberkennung möglich.

Im Gegensatz dazu erwiesen sich die Zusammensetzungen der Beispiele 39 bis 42 als äußerst empfindlich. Fig. 1 zeigt eine Schwarzweiß-Fotografie des Tests aus Beispiel 40 rund 10 min nach dem Auftrag der Zusammensetzung auf die Oberfläche (als der Schaum bereits begonnen hatte, von der Oberfläche abzufließen). Mit Bezugszeichen 1 ist der unverfärbt gebliebene, dünklere (tatsächlich violett gefärbte) Anteil des Schaums gekennzeichnet, mit 2 der hellere (tatsächlich grün gefärbte) Teil auf der stärker verunreinigten linken Hälfte (1 mg Glucose/100cm²) und mit 3 der hellere (grün gefärbte) Teil auf der weniger stark verunreinigten rechten Hälfte (0,1 mg Glucose/100cm²).

Man erkennt, dass die Verfärbung rechts zwar etwas weniger intensiv ausgeprägt, aber dennoch sehr gut mit freiem Auge erkennbar ist. Die Verunreinigung ist in beiden Fällen eindeutig detektierbar.

Die vorliegende Erfindung ist daher in der Lage, sogar organische Verunreinigungen in einer Menge von nur 0,1 mg/100cm² klar erkennbar, rasch und zuverlässig zu detektieren - und das, obwohl auch in dem in Fig. 1 gezeigten Beispiel 40 ein organisches Tensid als Verdickungsmittel eingesetzt wurde. Dieses lieferte offensichtlich über 10 min lang kein falsch-positives Ergebnis (da sich ansonsten der gesamte Schaum hätte verfärben müssen).

Geringere Mengen an Verunreinigungen können freilich auch nachgewiesen werden, sei es weiterhin mit freiem Auge oder auch unter Durchführung eines computerunterstützten digitalen Farbvergleichs. Dieser kann etwa erfolgen, indem die mit der Zusammensetzung bedeckte Oberfläche mit einer Digitalkamera fotografiert und das Foto mittels einer Farbvergleichssoftware mit einer vorgegebenen Farbskala verglichen wird, z.B. unter Umrechnung der Farben auf dem Foto, oder zumindest auf einem relevanten Ausschnitt desselben, in RGB-Werte.

Mittels der vorliegenden Erfindung können daher sogar Restverschmutzungen auf Oberflächen detektiert werden, die bereits einer Reinigung unterzogen wurden. Die vorliegende Erfindung eignet sich somit unter anderem hervorragend zur Qualitätskontrolle der Reinigungsvorgänge in Industriebetrieben.

### Beispiel 43 - nach unten weisende Oberfläche

Eine mit dem Schichtsilikat Laponite RD in einer Menge von 1,5 Gew.-% als Verdickungsmittel (analog zu Beispiel 41) gebildete gelförmige, thixotrope Zusammensetzung wurde auf die Unterseite einer Edelstahlplatte aufgesprüht, die analog zu den obigen Beispielen 39 bis 42 mit Glucose als organische Verunreinigung präpariert worden war. Wiederum trat binnen 30 s eine Grünfärbung auf und blieb während der folgenden 5 min sichtbar. Die Zusammensetzung zeigte während dieser Zeitspanne keinerlei Abtropfneigung.

### Beispiele 44 bis 58, Vergleichsbeispiele 84 und 85 - Braunsteinbildung

Zusammensetzungen wurden wie oben angegeben - jeweils mit Peroxodisulfat als zweitem Oxidationsmittel - mit den in nachstehender Tabelle 3 angeführten Arten und Mengen an Verdickungsmitteln hergestellt und auf ihre Braunsteinbildung untersucht.

Zu diesem Zweck wurden jeweils 100 ml einer Zusammensetzung - gegebenenfalls zusammen mit 1 ml einer 0,01%igen Glucoselösung als "Verunreinigung" - in eine transparente Glasflasche gefüllt, ein Metallplättchen aus Edelstahl wurde in die Flüssigkeit eingetaucht, die Flasche verschlossen und 20 min lang stehen gelassen. Danach wurde das Metallplättchen entnommen und genau wie die Flaschenwand visuell auf anhaftende Braunsteinrückstände untersucht. Die dabei beobachteten Mengen an anhaftendem Braunstein wurden wie folgt bewertet:

| | |
|---|---|
| - | keine Braunsteinablagerungen zu erkennen |
| + | minimale Braunsteinablagerungen |
| ++ | sichtbare Braunsteinablagerungen |
| +++ | starke Braunsteinablagerungen |
| ++++ | sehr starke Braunsteinablagerungen. |

Aus den Ergebnissen in umseitiger Tabelle 3 ist eindeutig zu erkennen, dass die Menge an Braunsteinablagerungen an der Flaschenwand und/oder am Metallplättchen in allen Beispielen der vorliegenden Erfindung gegenüber den Vergleichsbeispielen - mitunter deutlich - verringert werden konnte.

**Tabelle 3 - Braunsteinbildung**

| **Beispiel Nr.** | **Verdickungsmittel** | **Wirksubstanz** | + **Verunreinigung** | **VD (Gew.-%)** | **Bewertung: Wand** | **Bewertung: Plättchen** |
|---|---|---|---|---|---|---|
| V84 | - | - | nein | - | ++ | +++ |
| Beispiel 44 | Dowfax 2A1 | verzweigtes C12-Alkyldiphenyloxiddisulfonat | nein | 0,3 | ++ | - |
| Beispiel 45 | -"- | -"- | nein | 0,6 | + | + |
| Beispiel 46 | -"- | -"- | nein | 0,9 | ++ | + |
| Beispiel 47 | Laponite RD | synthetisches Schichtsilikat | nein | 5 | ++ | ++ |
| Beispiel 48 | -"- | -"- | nein | 10 | ++ | + |
| Beispiel 49 | -"- | -"- | nein | 15 | + | + |
| Beispiel 50 | HDK D1515B | pyrogene Kieselsäure | nein | 5 | +++ | +++ |
| Beispiel 51 | -"- | -"- | nein | 10 | ++ | ++ |
| Beispiel 52 | -"- | -"- | nein | 15 | + | - |
| V85 | - | - | ja | - | +++ | ++++ |
| Beispiel 53 | Dowfax 2A1 | verzweigtes C12-Alkyldiphenyloxiddisulfonat | ja | 0,3 | +++ | +++ |
| Beispiel 54 | -"- | -"- | ja | 0,6 | ++ | ++ |
| Beispiel 55 | -"- | -"- | ja | 0,9 | + | ++ |
| Beispiel 56 | Laponite RD | synthetisches Schichtsilikat | ja | 5 | +++ | +++ |
| Beispiel 57 | -"- | -"- | ja | 10 | ++ | ++ |
| Beispiel 58 | -"- | -"- | ja | 15 | + | - |

### Beispiele 59 bis 64 und Vergleichsbeispiele 86 und 87 - Indikatorwirkung

Um zu untersuchen, in welcher Menge Permanganat bei Reaktion mit organischen Verschmutzungen für einen gut detektierbaren Farbumschlag der Zusammensetzungen sorgt, wurden die folgenden Versuche durchgeführt.

Es wurden Zusammensetzungen hergestellt, die jeweils Folgendes enthielten:

| | |
|---|---|
| KMnO₄ | 0,01 g |
| HC | 1,3 g |
| NaOH | 0,35 g |
| VD | 0,9 g |
| Wasser | auf 1000 ml |

Als Verdicker (VD) kam in dieser Versuchsreihe in allen Fällen Laponite RD zum Einsatz. Zu diesen Lösungen, die somit eine Permanganat-Konzentration von 0,01 g/l enthielten, wurden unterschiedliche Mengen an zusätzlichem KMnO₄ zugesetzt, um die in nachstehender Tabelle 4 angegebenen Konzentrationen zu ergeben. Diese Lösungen wurden auf eine vertikale Keramikfliesenwand aufgesprüht, auf die zuvor analog zu den Beispielen 39 bis 42 eine 0,01%ige Glucoselösung aufgebracht und getrocknet worden war, um eine Glucosemenge von etwa 1 mg/100cm² als organische "Verunreinigung" der Oberfläche zu erhalten

Auf den mit den Zusammensetzungen besprühten Flächen wurde der Farbumschlag des Indikators von Violett zu Grün aufgrund der Reduktion des Permanganats visuell untersucht und wie folgt bewertet:

| | |
|---|---|
| - | Farbumschlag nicht oder kaum erkennbar |
| + | Farbumschlag schwer erkennbar |
| ++ | Farbumschlag gut erkennbar |

**Tabelle 4 - Farbumschlag**

| **Beispiel** | **Zusatz KMnO₄ (mg)** | **Konz. KMnO₄ (g/l)** | **Farbumschlag** |
|---|---|---|---|
| Vergleichsbeispiel 86 | 0 | 0,01 | - |
| Beispiel 59 | 100 | 0,11 | + |
| Beispiel 60 | 150 | 0,16 | ++ |
| Beispiel 61 | 200 | 0,21 | ++ |
| Beispiel 62 | 300 | 0,31 | ++ |
| Beispiel 63 | 500 | 0,51 | ++ |
| Beispiel 64 | 600 | 0,61 | + |
| Vergleichsbeispiel 87 | 750 | 0,76 | - |

Man erkennt, dass ab einer Permanganat-Konzentration von 0,11 g/l und bis zu einer Konzentration von 0,61 g/l ein zur Detektion von Verschmutzungsfreiheit prinzipiell geeigneter Farbumschlag erkennbar war. Gut erkennbar war dieser im Konzentrationsbereich von 0,16 bis 0,51 g/l. Bei 0,01 g/l Permanganat war die Färbung der Zusammensetzung zu blass, während jene bei 0,76 g/l zu intensiv war, um die Farbumschläge eindeutig feststellen zu können.

Daraus lässt sich ein bevorzugter Konzentrationsbereich für Permanganat als Indikator von etwa 0,1 bis 0,6 g/l und ein noch bevorzugterer Bereich von etwa 0,15 bis 0,5 g/l ableiten.

Neben den zu bevorzugenden Permanganat-Konzentrationsbereichen zeigen die obigen Beispiele aber auch klar, dass in solchen bevorzugten Ausführungsformen der vorliegenden Erfindung Permanganat eigentlich nicht als Oxidationsmittel, sondern vielmehr als Indikatorsystem zum Einsatz kommt, zumal das Ausbleiben eines Farbumschlags anzeigt, dass das neben Permanganat enthaltene Oxidationsmittel bereits sämtliche oxidierbaren Verunreinigungen beseitigt hat. Somit handelt es sich in diesen Ausführungsformen der Erfindung bei dem "zusätzlichen Oxidationsmittel" tatsächlich um das Hauptoxidationsmittel.

### Beispiel 65 - Reinigungswirkung

Unter Berücksichtigung der Ergebnisse der obigen Beispiele wurde das Verhältnis der Mengen an Permanganat und zusätzlichem Oxidationsmittel bzw. die Menge an Oxidationsmitteln in den Zusammensetzungen untersucht, die mindestens erforderlich ist, um binnen einer relativ kurzen Einwirkdauer neben der Detektionswirkung auch eine gründliche Reinigung der Oberflächen von hartnäckigen Verschmutzungen zu bewirken. Hierzu wurden die folgenden Mischungen hergestellt, wobei die Menge an Hypochlorit wie in nachstehender Tabelle 5 angeführt variiert wurde:

| | |
|---|---|
| KMnO₄ | 0,01 g |
| HC | zwischen 0,03 und 1,3 g |
| NaOH | 0,35 g |
| VD | 0,9 g |
| Wasser | auf 1000 ml |

Als schwer zu entfernende künstliche Verunreinigung wurden in dieser Versuchsreihe 15 ml eines Gemischs aus 40 g Malzextrakt und 8 g Kieselgur (in 38 g Stadtwasser) auf Stahlplatten von 15 x 15 cm mit bekanntem Gewicht aufgebracht und getrocknet. Darauf wurden in der Folge die obigen Mischungen aufgesprüht, durchwegs 2 min lang einwirken gelassen, danach mit 50 ml Wasser abgespült und getrocknet.

Die trockenen Platten wurden in der Folge ausgewogen, um zu detektieren, ob sich noch organische Rückstände darauf befanden.

In der nachstehenden Tabelle 5 wurde die Reinigungswirkung bezüglich der künstlichen Verunreinigung (insgesamt 48 g) anhand dessen bewertet, wie viel davon auf der jeweiligen Platte verblieben war:

| | |
|---|---|
| -- | > 30 g |
| - | < 30 g, aber > 20 g |
| + | < 20, aber > 10 g |
| ++ | < 10 g |

**Tabelle 5 - Reinigungswirkung**

| **Zusatz HC (g)** | **Verhältnis HC: KMn04** | **Reinigungswirkung** |
|---|---|---|
| 0,0 | - | -- |
| 0,03 | 3:1 | - |
| 0,05 | 5:1 | - |
| 0,10 | 10:1 | + |
| 0,20 | 20:1 | ++ |
| 0,30 | 30:1 | ++ |
| 0,50 | 50:1 | ++ |
| 1,30 | 130:1 | ++ |

Dabei zeigt sich, dass die Menge an "zusätzlichem" bzw. Hauptoxidationsmittel neben dem Permanganat-Indikator zumindest das 10fache der Permanganatmenge, noch besser zumindest das 20fache und im Hinblick auf besonders rasche Reinigung insbesondere zumindest das 30fache der Permanganatmenge, betragen sollte, sowie dass eine Konzentration von zumindest 0,3 g/l Oxidationsmittel vonnöten ist, um Oberflächen in sehr kurzer Zeit möglichst rückstandsfrei von schwer entfernbaren organischen Verunreinigungen befreien zu können.

Die obigen Beispiele zeigen somit, dass durch die Verwendung eines geeignet gewählten Verdickungsmittels in den Zusammensetzungen nicht nur die Fließfähigkeit für eine Zeitspanne von mehreren Minuten so weit eingeschränkt war, dass die Zusammensetzungen sogar an vertikalen und nach unten weisenden Oberflächen hafteten, sondern dass auch die Stabilität des Oxidationsmittels erhöht und die Braunsteinbildung wirksam unterdrückt werden konnte, was die Nachweisbarkeit organischer Verunreinigungen durch einfachen Farbumschlag verbessert und die Bildung anorganischer Rückstände auf den zu prüfenden Oberflächen deutlich reduziert. Darüber hinaus wurde bewiesen, dass bei geeigneter Wahl der Parameter überraschenderweise sogar organische Tenside als Verdickungsmittel in den Zusammensetzungen eingesetzt werden können. Bei Verwendung anorganischer Verdickungsmittel hingegen sind synthetischen gegenüber natürlichen Materialien zu bevorzugen, da letztere - wohl aufgrund der Gegenwart natürlicher organischer Verunreinigungen darin - zu falschen Ergebnissen bei der Detektion führen können.

## Patentansprüche

1. Verwendung einer Zusammensetzung auf wässriger Basis, die
a) zumindest ein Oxidationsmittel, das organische Verunreinigungen in einer vorbestimmten Zeitspanne oxidiert,
b) ein Farbindikatorsystem und
c) ein oder mehrere Verdickungsmittel
umfasst, zum Detektieren etwaiger Verschmutzung einer Oberfläche mit organischen Substanzen mittels Sichtprüfung nach flächigem Auftrag der Zusammensetzung auf die Oberfläche, wobei
- die Zusammensetzung nach dem flächigen Auftrag aufgrund der Wirkung des Verdickungsmittels für die genannte vorbestimmte Zeitspanne nicht abfließt oder abtropft;
- das oder die Verdickungsmittel während der vorbestimmten Zeitspanne in der Zusammensetzung stabil ist/sind; und
- das oder die Verdickungsmittel ausgewählt ist/sind aus synthetischen Schichtsilikaten, pyrogener Kieselsäure, Fettalkyl(benzol)sulfaten, -sulfonaten, -carboxylaten,
- phosphaten und -aminoxiden sowie Gemischen davon;
und wobei ein Farbumschlag der auf die Oberfläche aufgetragenen Zusammensetzung während der genannten vorbestimmten Zeitspanne organische Verschmutzungen der Oberfläche an dieser Stelle anzeigt.

2. Verwendung nach Anspruch 1, **dadurch gekennzeichnet, dass** die vorbestimmte Zeitspanne zumindest 10 s beträgt.

3. Verwendung nach Anspruch 1 oder 2, **dadurch gekennzeichnet, dass** das zumindest eine Oxidationsmittel aus Permanganaten, Peroxodisulfaten, Salzen von Halogensauerstoffsäuren und Gemischen davon ausgewählt ist.

4. Verwendung nach einem der vorangegangenen Ansprüche, **dadurch gekennzeichnet, dass** das Oxidationsmittel Permanganat umfasst.

5. Verwendung nach Anspruch 4, **dadurch gekennzeichnet, dass** das Oxidationsmittel neben Permanganat zumindest ein zusätzliches Oxidationsmittel umfasst, dessen Oxidationspotenzial über jenem von Permanganat liegt.

6. Verwendung nach Anspruch 5, **dadurch gekennzeichnet, dass** das zusätzliche Oxidationsmittel aus Peroxodisulfat und Hypochlorit ausgewählt ist.

7. Verwendung nach einem der Ansprüche 4 bis 6, **dadurch gekennzeichnet, dass** das Permanganat gleichzeitig als Farbindikator dient.

8. Verwendung nach einem der Ansprüche 4 bis 7, **dadurch gekennzeichnet, dass** organische Verdickungsmittel in einer Gesamtmenge von nicht mehr als 2 Gew.-% der gesamten Zusammensetzung enthalten sind.

9. Verwendung nach Anspruch 8, **dadurch gekennzeichnet, dass** organische Verdickungsmittel in einer Gesamtmenge von nicht mehr als 1 Gew.-% der gesamten Zusammensetzung enthalten sind.

10. Verwendung nach einem der vorangegangenen Ansprüche, **dadurch gekennzeichnet, dass** die Zusammensetzung weiters ein oder mehrere Hilfsmittel, ausgewählt aus pH-Reglern, Härtestabilisatoren und Bioziden enthält.

11. Verwendung nach einem der vorangegangenen Ansprüche, **dadurch gekennzeichnet, dass** die Sichtprüfung computerunterstützt unter Durchführung digitaler Farbvergleiche durchgeführt wird.

## Claims

1. Use of a water-based composition comprising
a) at least one oxidant that oxidizes organic impurities within a predetermined period of time,
b) a color-indicator system, and
c) one or more thickeners
for detecting any contamination of a surface by organic substances by means of visual inspection after an aerial application of the composition to the surface, wherein
- the composition does not flow off or drip off for said predetermined period of time after aerial application thereof due to the effect of the thickener;
- the one or more thickeners is/are stable within the composition during the predetermined period of time; and
- the one or more thickeners is/are selected from synthetic phyllosilicates, fumed silica, and fatty alkyl (benzene) sulfates, sulfonates, carboxylates, phosphates and aminoxides, as well as mixtures thereof;
and wherein a color change of the composition applied to the surface during said predetermined period of time indicates organic contaminations of the surface at that location.

2. The use according to claim 1, **characterized in that** the predetermined period of time is at least 10 s.

3. The use according to claim 1 or 2, **characterized in that** the at least one oxidant is selected from permanganate, peroxodisulfate, salts of halogen oxoacids and mixtures thereof.

4. The use according to any of the preceding claims, **characterized in that** the oxidant comprises permanganate.

5. The use according to claim 4, **characterized in that**, in addition to permanganate, the oxidant comprises at least one additional oxidant, the oxidation potential of which is higher than that of permanganate.

6. The use according to claim 5, **characterized in that** the additional oxidant is selected from peroxodisulfate and hypochlorite.

7. The use according to any one of claims 4 to 6, **characterized in that** the permanganate at the same time serves as color indicator.

8. The use according to any one of claims 4 to 7, **characterized in that** organic thickeners are contained in a total amount of not more than 2 % by weight of the entire composition.

9. The use according to claim 8, **characterized in that** organic thickeners are contained in a total amount of not more than 1 % by weight of the entire composition.

10. The use according to any one of the preceding claims, **characterized in that** the composition additionally contains one or more auxiliary agents selected from pH regulators, hardness stabilizers, and biozides.

11. The use according to any one of the preceding claims, **characterized in that** the visual inspection is computer-assisted and comprises the conduction of digital color comparisons.

## Revendications

1. Usage d'une composition aqueuse, comportant
a) au moins un agent oxydant apte à oxyder des contaminations organiques dans un laps de temps prédéfini,
b) un système d'indicateurs de couleur; et
c) un ou plusieurs agents épaississants
pour détecter d'éventuelles contaminations d'une surface avec des substances organiques par vérification visuelle après vaste application de la composition sur la surface,
- la composition ne s'écoulant ou ne s'égouttant pas dans le laps de temps prédéfini mentionné, après avoir été appliquée vastement sur la surface, grâce à l'action de l'agent épaississant;
- le ou les agents épaississants étant stables dans la composition pendant le laps de temps prédéfini; et
- le ou les agents épaississants étant choisis parmi des silicates en couches, de l'acide silicique pyrogéné, des (benzène)sulfates, sulfonates, carboxylates, phosphates et oxydes d'amine d'alkyle gras, ainsi que des mélanges de ceux-ci;
et un changement de couleur de la composition appliquée sur la surface indiquant, pendant le laps de temps prédéfini mentionné, des contaminations organiques de la surface à cette position.

2. Usage selon la revendication 1, **caractérisé en ce que** le laps de temps prédéfini est d'au moins 10 s.

3. Usage selon la revendication 1, **caractérisé en ce que** l'au moins un agent oxydant est choisi parmi des permanganates, des peroxodisulfates, des sels des oxo-acides d'halogéne et des mélanges de ceux-ci.

4. Usage selon l'une quelconque des revendications précédentes, **caractérisé en ce que** l'agent oxydant comprend du permanganate.

5. Usage selon la revendication 4, **caractérisé en ce que**, en plus du permanganate, l'agent oxydant comprend au moins un agent oxydant additionnel, dont le potentiel oxydant est supérieur à celui du permanganate.

6. Usage selon la revendication 5, **caractérisé en ce que** l'agent oxydant additionnel est choisi parmi le peroxidisulfate et l'hypochlorite.

7. Usage selon l'une des revendications 4 à 6, **caractérisé en ce qu'**en même temps le permanganate sert d'indicateur de couleur.

8. Usage selon l'une des revendications 4 à 7, **caractérisé en ce que** les agents épaississants sont présents en une quantité totale de pas plus que 2 % en poids de la totalité de la composition.

9. Usage selon la revendication 8, **caractérisé en ce que** les agents épaississants sont présent en une quantité totale de pas plus qu'1% en poids de la totalité de la composition.

10. Usage selon l'une quelconque des revendications précédentes, **caractérisé en ce que**, de plus, la composition comprend un ou plusieurs adjuvants, choisis parmi des régulateurs de pH, des stabilisateurs de dureté et des biocides.

11. Usage selon l'une quelconque des revendications précédentes, **caractérisé en ce que** la vérification visuelle est exécutée de façon assistée par ordinateur et par l'exécution de comparaisons numériques des couleurs.
